# NOUVEAU FASCICULE DE BREVET EUROPEEN

(11) **EP 0 590 689 B2**
(45) Date de publication et mention de la décision concernant l'opposition: **16.08.2006**
(45) Mention de la délivrance du brevet: 22.05.2002
(21) Numéro de dépôt: 93116225.9
(22) Date de dépôt: 17.06.1985
(51) Int. Cl.: C12N 15/11, C12P 21/00, C07K 2/00, G01N 33/48, A61K 38/02, A61K 39/00, A61K 39/29

(54) **Procédé d'obtention d'ADN, ARN, peptides, polypeptides ou protéines, par une technique de recombinaison d'ADN**
Verfahren zum Erhalten von DNS, RNS, Peptiden, Polypeptiden oder Proteinen durch DMS-Rekombinant-Verfahren
Method for obtaining DNA, RNA, peptides, polypeptides or proteins by means of a DNA-recombinant technique

(30) Priorité: 30.03.1985 CH 137985
(43) Date de publication de la demande: 06.04.1994
(62) Demande divisionnaire de: 85902946.4
(73) Titulaire: KAUFFMAN, Stuart A., Santa Fe, NM 87505 (US)
(72) Inventeur: Ballivet, Marc, 1208 Genève (CH); Kauffman, Stuart A., Santa Fe, New Mexico 87505 (US)
(74) Mandataire: Fisher, Adrian John

(56) Documents cités:
- DE-A- 3 246 071
- DE-A- 3 300 632
- DE-A- 3 303 173
- US-A- 5 432 018
- CHEMICAL ABSTRACTS, vol. 84, no. 5, 2 Février 1976, Columbus, Ohio, US; abstract no. 26960x, G.W. HOFFMANN 'Stochastic theory of the origin of the genetic code.' page 136 ; & ANNU. REV.PHYS.CHEM. vol. 26 , 1975 page 123-144
- Devlin JJ et al., "Random Peptide Libraries: A Source of Specific Protein Binding Molecules", Science, vol. 249, 27.07.1990, p. 404-4406
- Cwirla SE et al., "Peptides on phage: A vast library of peptides for identifying ligands", Proc Natl Acad Sci USA, vol. 87, 08.1990, p. 6378-6382
- Maniatis et al., "Molecular Cloning: A Laboratory Manual", 1982, p. 99, 239-240, 473
- Conn MM et al., "Porphyrin Metalation Catalyzed by Small RNA Molecule", J Am Chem Soc, vol 118, 1996, 7012-7013
- Ballinger MD et al., "Selection of Heregulin Variants Having Higher Affinity for the ErbB3 Receptor by Monovalent Phage Display", Journal of Biological Chemistry, vol. 273, no. 19, 8.5.1998, p. 11675-11684
- O'Neil KT et al., "Identification of Novel Peptide Antagonists for GPIIb/IIIa From a Conformationally Constrained Phage Peptide Library", Proteins, vol. 14, 1992, p. 509-515
- Cull MG et al., "Screeining for receptor ligands using large libraries of peptides linked to the C terminus of the lac repressor", Proc Natl Acad Sci USA, vol. 89, 03.1992, p. 1865-1869
- Clackson T and Wells JA, "In vitro selection from protein and peptide libraries", Tibtech, vol. 12, 05.1994, p. 173-184

## Description

La présente invention a pour objet un procédé d'obtention d'ADN, ARN, peptides, polypeptides ou protéines, au moyen de cellules-hôtes modifiées contenant des.gènes capables d'exprimer ces ARN, peptides, polypeptides ou protéines, c'est-à-dire en utilisant une technique de recombinaison d'ADN.

L'invention vise notamment à produire des gênes ou fragments de gènes produits de manière stochastique, de façon à permettre l'obtention simultanée, après transcription et traduction de ces gènes, d'un très grand nombre de protéines complètement nouvelles ou hybrides de protéines connues, en présence de cellules-hôtes (souches bactériennes ou eucaryotes) contenant les gènes respectivement capables d'exprimer ces protéines et d'effectuer ensuite une sélection ou criblage parmi lesdites souches, en vue de déterminercelles qui produisent des protéines présentant des propriétés désirées, par exemple des propriétés structurelles enzymatiques, catalytiques, antigéniques, pharmacologiques, ou des propriétés de ligand, et plus généralement, des propriétés chimiques, biochimiques, biologiques, etc.

L'invention a également pour but de permettre l'obtention de séquences d'ADN ou d'ARN présentant des propriétés utilisables, notamment des propriétés chimiques, biochimiques ou biologiques.

On comprend donc que l'invention est susceptible de recevoir de très nombreuses applications, dans des domaines très variés de la science, de l'industrie et de la médecine.

L'invention pourvoit une méthode d'identification d'un peptide, d'un polypeptide ou d'un protéine qui se lie à un ligand selon revendication 1. L'invention pourvoit d'autres méthodes selon les revendications 9, 10, 11, 12, 18 et 19.

Conformément à un premier mode de mise en oeuvre des procédés de l'invention, on produit les gènes par copolymérisation stochastique des quatre sortes de deoxyphosphonucléotides A, C, G et T à partir des deux extrémités d'un vecteur d'expression préalablement linéarisé, puis formation d'extrémités cohésives de façon à former un premier brin d'ADN produit de manière stochastique, constitué d'une molécule du vecteur d'expression possédant deux séquences produits de manière stochastique dont les extrémités 3' sont complémentaires, suivie de la synthèse du second brin de cet ADN produit de manière stochastique.

Selon un deuxième mode de mise en oeuvre, on produit les gènes par copolymérisation stochastique d'oligonucléotides sans extrémités cohésives, de façon à former des fragments d'ADN produits de manière stochastique, suivie de la ligation de ces fragments à un vecteur d'expression préalablement linéarisé.

Le vecteur d'expression peut être un plasmide, notamment un plasmide bactérien. D'excellents résultats ont été obtenus en utilisant, comme vecteur d'expression, le plasmide pUC8.

Le vecteur d'expression peut également être un ADN viral ou un hybride de plasmide et d'ADN viral.

Les cellules-hôtes peuvent être des cellules procaryotes telles que les cellules HB 101 et C 600, ou des cellules eucaryotes.

Lorsque l'on procède conformément au deuxième mode de mise en oeuvre susmentionné, on peut utiliser des oligonucléotides formant un groupe d'octamères palindromiques.

Des résultats particulièrement bons sont obtenus en utilisant le groupe d'octamères palindromiques suivant : 
5' GGAATTCC 3'
5' GGTCGACC 3'
5' CAAGCTTG 3'
5' CCATATGG 3'
5' CATCGATG 3'
 On peut également utiliser des oligonucléotides formant un groupe d'heptamères palindromiques.

De très bons résultats sont obtenus en utilisant le groupe d'heptamères palindromiques suivant: 
5' XTCGCGA 3'
5' XCTGCAG 3'
5' RGGTACC 3'
 où X = A, G, C ou T et R = A ou T.

Conformément à un mode de mise en oeuvre particulièrement avantageux, l'on isole et purifie l'ADN transformant des plasmides provenant d'une culture de souches indépendantes de cellules-hôtes modifiées obtenues en procédant de la manière spécifiée ci-dessus, puis l'on provoque la coupure de l'ADN purifié au moyen d'au moins un enzyme de restriction correspondant à un site spécifique de coupure enzymatique présent dans ces octamères ou heptamères palindromiques mais absent du vecteur d'expression utilisé, cette coupure étant suivie de l'inactivation de l'enzyme de restriction, et l'on traite ensuite simultanément l'ensemble des fragments d'ADN produits de manière stochastique linéarisés, ainsi obtenus, par la T4 DNA ligase, de façon à créer un nouvel ensemble d'ADN contenant des séquences produits de manière stochastique nouvelles, ce nouvel ensemble pouvant donc contenir un nombre de gènes produits de manière stochastique supérieur au nombre de gènes de l'ensemble initial, et on utilise ce nouvel ensemble d'ADN transformant pour modifier des cellules-hôtes et cloner les gènes, et, finalement, on crible et/ou on sélectionne et on isole les nouvelles souches de cellules-hôtes transformées et finalement, on les cultive de façon à produire au moins un peptide ou polypeptide, par exemple une protéine nouvelle.

La propriété servant de critère de sélection des souches de cellules-hôtes peut être l'aptitude des peptides ou polypeptides, produits par cette souche, à se lier à un ligand donné.

L'invention a également pour objet l'utilisation du peptide ou polypeptide obtenu par le procédé spécifié ci-dessus pour la détection et/ou la titration d'un ligand.

Conformément à un mode de mise en oeuvre particulièrement avantageux, le critère de sélection de la souche de cellules-hôtes modifiée est l'aptitude des peptides ou polypeptides à simuler ou modifier les effets d'une molécule biologiquement active, par exemple une protéine, et l'on effectue le criblage et/ou la sélection de la souche de cellules-hôtes modifiée produisant au moins un peptide ou polypeptide ayant cette propriété en préparant des anticorps contre cette molécule active, et en utilisant ces anticorps, après leur purification, pour identifier les souches contenant ce peptide ou polypeptide, puis en cultivant les souches ainsi identifiées et en séparant et en purifiant le peptide ou polypeptide produit par ces souches, et, finalement, en soumettant ce peptide ou polypeptide à un essai in vitro pour vérifier qu'il présente bien l'aptitude à simuler ou modifier les effets de ladite molécule.

Conformément à un autre mode de mise en oeuvre du procédé selon l'invention, la propriété servant de critère de sélection est d'avoir au moins un épitope semblable à l'un des épitopes d'un antigène donné.

Selon une variante du procédé, l'on identifie et isole les souches de cellules-hôtes modifiées produisant les peptides ou polypeptides ayant la propriété désirée par chromatographie d'affinité sur anticorps correspondant à une protéine exprimée par la partie naturelle de l'ADN hybride.

Par exemple, dans le cas où la partie naturelle de l'ADN hybride contient un gêne exprimant la β-galactosidase, l'on peut avantageusement identifier et isoler lesdites souches de cellules-hôtes modifiées par chromatographie d'affinité sur anticorps anti-β-galactosidase.

Après expression et purification des peptides
ou polypeptides hybrides, on peut séparer et isoler leurs parties nouvelles.

L'invention porte également sur une application du procédé spécifié ci-dessus pour la préparation d'un vaccin, cette application étant caractérisé par le fait que l'on isole des anticorps contre un agent pathogène, par exemple des anticorps formés après injection de cet agent pathogène dans l'organisme d'un animal capable de former des anticorps contre cet agent, et on utilise ces anticorps pour identifier les clones produisant au moins une protéine ayant au moins un épitope semblable à l'un des épitopes de l'agent pathogène, on cultive les souches de cellules-hôtes modifiées correspondant à ces clones, de façon à produire cette protéine, on isole et purifie cette protéine à partir des cultures de ces souches de cellules, et on utilise cette protéine pour la production d'un vaccin contre l'agent pathogène.

Par exemple, pour la préparation d'un vaccin anti-HVB, l'on peut extraire et purifier au moins une protéine de capside de virus HVB, injecter cette protéine dans l'organisme d'un animal capable de former des anticorps contre cette protéine, recueillir et purifier les anticorps ainsi formés, utiliser ces anticorps pour identifier les clones produisant au moins une protéine ayant au moins un épitope semblable à l'un des épitopes du virus HVB, cultiver les souches de cellules-hôtes modifiées correspondant à ces clones, de façon à produire cette protéine, isoler et purifier cette protéine à partir des cultures de ces souches de cellules, et utiliser cette protéine pour la production d'un vaccin anti-HVB.

Avantageusement, conformément à un mode de mise en oeuvre du procédé selon l'invention, les cellules-hôtes consistent en bactéries du genre Escherichia coli dont le génome ne contient ni le gène naturel exprimant la β-galactosidase, ni le gène EBG, c'est-à-dire des bactéries E. coli (Z-, EBG⁻), on cultive les cellules-hôtes modifiées en présence du milieu X-gal et de l'inducteur IPTG, que l'on détecte, dans le milieu de culture, les clones positifs pour la fonction β-galactosidase, et, finalement, on transplante ensuite cet ADN dans une souche de cellules-hôtes appropriée à la culture en grande quantité en vue de la production industrielle d'au moins un peptide ou polypeptide.

La propriété servant de critère de sélection des souches de cellules-hôtes transformées peut également être l'aptitude, des polypeptides ou protéines, produites par culture de ces souches, à se lier à un composé donné.

Ce composé peut être, notamment, avantageusement choisi parmi les peptides, les polypeptides et les protéines, notamment les protéines régulatrices de l'activité de transcription de l'ADN.

D'autre part, ledit composé peut également être choisi parmi les séquences d'ADN et d'ARN.

L'invention a également pour objet les protéines obtenues dans le cas où la propriété servant de critère de sélection des souches de cellules-hôte transformées consiste précisément dans l'aptitude de ces protéines à se lier à des protéines régulatrices de l'activité de transcription de l'ADN, ou encore à des séquences d'ADN ou d'ARN.

L'invention a, en outre, pour objet l'utilisation d'une protéine, obtenue dans le premier cas particulier qui vient d'être mentionné, comme séquence cis-régulatrice de la réplication ou la transcription d'une séquence d'ADN voisine.

D'autre part, l'invention a également pour objet l'utilisation des protéines obtenues dans le second cas particulier susmentionné pour modifier les propriétés de transcription ou de réplication d'une séquence d'ADN, dans une cellule contenant cette séquence d'ADN, et exprimant cette protéine.

L'invention a également pour objet un procédé de production d'ADN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on effectue un criblage et/ou une sélection de cet ensemble, de façon à identifier les cellules hôtes qui renferment dans leur génome des séquences stochastiques d'ADN présentant au moins une propriété désirée, et l'on isole finalement l'ADN à partir de cultures des cellules-hôtes ainsi identifiées.

L'invention a, en outre, pour objet un procédé de production d'ARN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on cultive simultanément les souches indépendantes de cellules-hôtes modifiées ainsi produites, que l'on effectue un criblage et/ou une sélection de cet ensemble, de façon à identifier les cellules-hôtes qui renferment des séquences stochastiques d'ARN présentant au moins une propriété désirée, et que l'on isole l'ARN à partir de cultures de cellules-hôtes ainsi identifiées.

Ladite propriété peut être, l'aptitude à se lier à un composé donné, qui peut être, par exemple, un peptide, un polypeptide ou une protéine.

On va maintenant décrire, plus en détails, le procédé selon l'invention, ainsi que certaines de ses applications, en se référant à des exemples, non limitatifs, de mise en oeuvre.

Tout d'abord, on va décrire des modes opératoires particulièrement avantageux pour effectuer la synthèse de gènes produits de manière stochastique et l'introduction de ces gènes dans des bactéries, de manière à produire des souches de bactéries transformées.

### I) Synthèse directe sur un vecteur d'expression

### a) Linéarisation du vecteur

30 µg (c'est-à-dire approximativement 10¹³ molécules) du vecteur d'expression pUC8 sont linéarisés par incubation pendant 2 heures à 37°(avec 100 unités de l'enzyme de restriction Pstl dans une volume de 300 µl du tampon standard approprié. Le vecteur linéarisé est traité au phénol-chloroforme puis précipité à l'éthanol, repris dans un volume de 30 µl et chargé sur un gel d'agarose à 0,8 %, en milieu standard TEB. Après migration dans un champ de 3V/cm pendant trois heures, le vecteur linéaire est électro-élué, précipité à l'éthanol et repris dans 30 µl d'eau.

### b) Synthèse stochastique par l'enzyme

### Terminal-Transférase (TdT)

On fait réagir 30 µg du vecteur linéarisé avec 30 unités de TdT dans 300 µl du tampon approprié, pendant deux heures, à 37°C, en présence de 1 mM de dGTP, 1mM de dCTP, 0,3mM de dTTP et 1 mM de dATP: On choisit une concentration plus basse de dTTP, dans le but de diminuer la fréquence des codons "stop" dans l'ARN messager correspondant. Un résultat semblable, quoique moins favorable, peut être obtenu en utilisant une concentration plus basse pour le d'ATP que pour les autres deoxynucléotides triphosphates.Le progrès de la réaction de polymérisation sur l'extrémité 3' des sites Pst I est suivi par l'analyse sur gel de parties aliquotes prélevées en cours de réaction.

Lorsque la réaction atteint ou dépasse une valeur moyenne de 300 nucléotides par extrémité 3', elle est interrompue et les nucléotides libres sont séparés du polymère par précipitation différentielle ou par passage sur tamis moléculaire du type Biogel P60. Après concentration par précipitation à l'éthanol, le polymère est soumis séquentiellement à une polymérisation supplémentaire par TdT en présence de dATP d'abord, puis de dTTP. Ces deux dernières réactions sont séparées par une filtration sur gel et conduites pendant des temps courts (30 secondes à 3 minutes), de manière à aboutir à l'addition séquentielle de 10-30A, suivis de 10-30 T à l'extrémité 3' des polymères.

### c) Synthèse du second brin de l'ADN produit de manière stochastique

Chaque molécule de vecteur possède, à l'issue des opérations précédentes, deux séquences stochastiques dont les extrémités 3' sont complémentaires. Le mélange de polymères est alors incubé dans des conditions favorisant l'hybridation de ces extrémités complémentaires (150 mM Nacl, 10 mM Tris-HCl, pH 7,6, 1 mM EDTA, à 65°C, pendant 10 minutes, puis abaissement de la température jusqu'à 22°C, à raison de 3 à 4°C/heure). Les polymères hybridés sont alors soumis à l'action de 60u du grand fragment de la polymérase 1 (Klenow) en présence des quatre nucléotides triphosphates (200mM) à 4°C, pendant deux heures. Cette étape réalise la synthèse du second brin à partir de l'extrémité 3' des polymères hybridés. Les molécules résultant de cette synthèse directe à partir d'un vecteur linéarisé sont alors utilisées pour transformer des cellules compétentes.

### d) Transformation des souches compétentes

100 à 200 ml de cellules compétentes HB101 ou C600, à la concentration de 10¹⁰ cellules/ml, sont incubés avec la préparation d'ADN produit de manière stochastique, en présence de 6mM CaCl₂, 6 mM Tris-Hcl, pH8, 6 mM MgCl₂, pendant 30 minutes, à 0°C. Un choc thermique de 3 minutes à 37°C est imposé au mélange, suivi par l'addition de 400-800 ml de milieu de culture NZY, sans antibiotique. La culture transformée est incubée à 37°C pendant 60 minutes, puis diluée à 10 litres par addition de milieu NZY contenant 40 µg/ml d'ampicilline. Après 3 à 5 heures d'incubation à 37°C, la culture amplifiée est soumise à une centrifugation, et le culot des cellules transformées est lyophilysé et conservé à -70°C. Une telle culture comprend de 3 x 10⁷ à 10⁸ transformants indépendants, contenant chacun un gène produit de manière stochastique unique inséré dans un vecteur d'expression.

### II) Synthèse de gènes produits de manière stochastique à partir d'oligo-nucléotides sans extrémités cohésives

Ce mode opératoire est fondé sur le fait que la polymérisation d'oligonucléotides palindromiques judicieusement choisis permet d'assembler des gènes produits de manière stochastique ne comportant aucun codon "stop" dans chacun des six cadres de lecture possibles, tout en assurant une représentation équilibrée de triplets spécifiant tous les acides aminés. Par ailleurs, et pour éviter la répétition de motifs de séquences dans la protéine résultante, les oligonucléotides penvent comporter un nombre de bases qui n'est pas un multiple de trois. L'exemple suivant décrit l'utilisation d'une combinaison possible d'oligonucléotides qui remplit ces critères :

### a) Choix d'un groupe d'octamères

Le groupe d'oligonucléotides suivant : 
5' GGAATTCC 3'
5' GGTCGACC 3'
5' CAAGCTTG 3'
5' CCATATGG 3'
5' CATCGATG 3'
 est composé de 5 palindromes (donc autocomplémentaires) dont il est facile de vérifier que leur polymérisation stochastique n'engendre pas de codon "stop" et spécifie tous les acides aminés.

Bien entendu, on pourrait également utiliser d'autres groupes d'octamères palindromiques n'engendrant pas de codons "stop" et spécifiant tous les acides aminés des polypeptides. On comprendra également que l'on pourrait utiliser des groupes d'octamères non palindromiques à condition d'employer aussi leurs compléments par la formation de segments bicaténaires.

### b) Assemblage d'un gène produit de manière stochastique à partir d'un groupe d'octamères

On fait réagir un mélange comportant 5 µg de chacun des oligonucléotides indiqués ci-dessus (préalablement phosphorylés en 5' par un procédé connu en soi) dans un volume de 100 µl contenant 1 mM ATP, 10 % de polyéthylèneglycol et 100 u de T₄ DNA ligase, dans le tampon approprié, à 13°C, pendant six heures. Cette étape effectue la polymérisation stochastique des oligomères sous forme bi-caténaire, sans extrémités cohésives. On isole alors par passe sur tamis moléculaire (Biogel P60) les polymères résultant de l'assemblage de 20 à 100 oligomères. Après concentration, cette fraction est à nouveau soumise à la polymérisation catalysée par la T4 DNA ligase dans les conditions décrites ci-dessus. On isole alors, comme décrit plus haut, les polymères résultant de l'assemblage d'au moins 100 oligomères.

### c) Préparation du plasmide hôte

Le vecteur d'expression pUC8 est linéarisé par l'enzyme Sma I dans le tampon approprié, comme décrit ci-dessus. Le vecteur linéarisé par l'enzyme Sma I ne comporte pas d'extrémités cohésives. Le vecteur linéarisé est alors traité par la phosphatase alcaline d'intestin de veau (CIP) à raison d'une unité par µg de vecteur dans le tampon approprié, à 37°C, pendant 30 minutes. L'enzyme CIP est alors inactivée par deux extractions successives au phénol-chloroforme. Le vecteur linéarisé et déphosphorylé est précipité à l'éthanol puis repris dans l'eau à 1 mg/ml.

### d) Ligation des gènes produits de manière stochastique au vecteur

Des quantités équimolaires de vecteur et de polymère sont mélangées et incubées en présence de 1000 u de T4 DNA ligase, 1 mM ATP, 10 % de polyéthylène glycol dans le tampon approprié, pendant 12 heures, à 13°C. Cette étape réalise la ligation des polymères dans le vecteur d'expression et engendre des molécules bicaténaires, circulaires et, par conséquent, transformantes.

### Transformation des souches compétentes

On procède à la transformation des souches compétentes de la manière précédemment décrite.

### III) Assemblage d'un gène produit de manière stochastique à partir d'un groupe d'heptamères

Ce mode opératoire se distingue de celui qui vient d'être décrit par le par le fait qu'il utilise des heptamères palindromiques comportant une extrémité cohésive variable, au lieu d'octamères. Il présente l'avantage de permettre l'assemblage de séquences produits de manière stochastique comportant une plus faible proportion de motifs identiques.

### a) Choix d'un groupe d'heptamères

On peut utiliser, par exemple, les 3 heptamères palindromiques suivants : 
5' XTCGCGA 3'
5' XCTGCAG 3'
5' RGGTACC 3'
 pour lesquels X = A, G, C ou T et R = A ou T et dont la polymérisation ne peut engendrer de codons "stop" et comporte des triplets spécifiant tous les acides aminés.

Bien entendu, on pourrait également utiliser tout autre groupe d'heptamères remplissant ces mêmes conditions.

### b) Polymérisation d'un groupe d'heptamères

Cette polymérisation s'effectue exactement de la manière précédemment décrite dans le cas des octamères.

### c) Elimination des extrémités cohésives

Les polymères ainsi obtenus comportent une base non appariée à leur deux extrémités 5'. Il est donc nécessaire d'additionner la base complémentaire aux extrémités 3' correspondantes. Ceci s'effectue de la manière suivante : on fait réagir 10 µg de polymères double brin avec 10 u d'enzymes de Klenow, en présence des quatre déoxynucléotidephosphates (200 mM) dans un volume de 100 µl, à 4°C, pendant 60 minutes. L'enzyme est inactivée par extraction au phénol-chloroforme et les polymères sont débarrassés des nucléotides libres résiduels par précipitation différentielle. Les polymères sont alors ligasés au plasmide-hôte (préalablement linéarisé et déphosphorylé) en procédant de la manière décrite ci-dessus.

Il est à remarquer que les deux derniers modes opératoires qui viennent d'être décrits utilisent des octamères ou heptamères palindromiques qui constituent des sites spécifiques d'enzymes de restriction. Ces sites sont absents pour la plupart du vecteur d'expression pUC8. II est donc possible d'augmenter considérablement la complexité d'une préparation initiale de gènes stochastiques en procédant de la manière suivante : on prépare l'ADN des plasmides provenant d'une culture de 10⁷ transformants indépendants obtenus par l'un des deux derniers modes opératoires décrits ci-dessus. A cet ADN purifié, on fait alors subir une digestion partielle par l'enzyme de restriction Cla I (mode opératoire II) ou par l'enzyme de restriction Pst I (mode opératoire III). Après inactivation de l'enzyme, l'ADN partiellement digéré est traité à la T4 DNA ligase, ce qui a pour effet de créer un nombre extrêmement grand de séquences nouvelles qui conservent les propriétés fondamentales des séquences initiales. Ce nouvel ensemble de séquences produits de manière stochastique est alors utilisé pour transformer des cellules compétentes.

Par ailleurs, les gènes produits de manière stochastique clonés en procédant conformément aux modes opératoires II et III peuvent être excisés intacts du vecteur d'expression pUC8 en utilisant les sites de restriction propres au vecteur de clonage et non représenté dans l'ADN produit de manière stochastique.

La recombinaison au sein des gènes produits de manière stochastique engendrés par les deux derniers modes opératoires qui viennent d'être décrits, recombinaison qui résulte de l'homologie interne et des motifs de récurrence, fournit une importante méthode additionnelle de mutagenèse in vivo des séquences codantes. Il en résulte une augmentation du nombre des gènes nouveaux qui peuvent être examinés.

Finalement, pourtout processus de génération de gènes synthétiques nouveaux, on peut utiliser de nombreuses techniques usuelles de modification des gènes in vivo ou in vitro, telles que changement du cadre de lecture, inversion des séquences par rapport à leur promoteur, ou utilisation de souches-hôtes exprimant un ou plusieurs tRNA suppresseurs.

En se référant à la partie de la description qui precède, on comprendra qu'il est possible de construire in vitro un nombre extrêmement grand (par exemple, supérieur à un milliard) de gènes différents, par polymérisation enzymatique de nucléotides et d'oligonucléotides. Cette polymérisation s'effectue selon un processus stochastique déterminé par les concentrations respectives des nucléotides ou oligonucléotides présents dans le milieu de réaction.

Comme indiqué ci-dessus, deux méthodes peuvent être utilisées pour cloner de tels gènes (ou séquences codantes) : la polymérisation peut s'effectuer directement sur un vecteur de clonage d'expression, préalablement linéarisé, ou bien on peut choisir de procéder séquentiellement à la polymérisation puis à la ligation des polymères au vecteur de clonage et d'expression.

Dans les deux cas, on procède ensuite à la transformation ou à la transfection de cellules bactériennes compétentes (ou de cellules en culture). Cette étape réalise le clonage des gènes stochastiques dans des cellules vivantes où ils sont indéfiniment propagés et exprimés:

Bien entendu, outre les modes opératoires qui viennent d'être décrits, on pourrait également employer toute autre méthode appropriée pour la synthèse de séquences produits de manière stochastiques. En particulier, on pourrait procéder à la polymérisation, par voie biochimique, d'oligomères monocaténaires d'ADN ou d'ARN obtenus par synthèse chimique, puis traiter ces segments d'ADN ou d'ARN par des procédés connus en soi de préparation de copies d'ADN (c ADN) bicaténaire en vue du clonage des gènes.

### Criblage ou sélection des souches de cellules-hôtes modifiées

L'étape ultérieure du procédé selon l'invention consiste à examiner les cellules transformées ou transfectées, par sélection ou criblage, en vue d'isoler une ou plusieurs cellules dont l'ADN transformant ou transfectant conduise la synthèse d'un produit de transcription (ARN) ou de traduction (protéine) possédant une propriété souhaitée. Ces propriétés peuvent être, par exemple, enzymatiques, fonctionnelles ou structurelles.

Une des particularités les plus remarquables du procédé selon l'invention est de permettre le criblage ou la sélection simultanée d'un produit exploitable (ARN ou protéine) et de son gène producteur. De surcroît, l'ADN synthétisé et cloné comme décrit peut être sélectionné ou criblé en vue d'isoler des séquences d'ADN constituant un produit en soi, doté de propriétés biochimiques exploitables.

On va maintenant décrire, à titre d'exemples non limitatifs, des modes opératoires préférentiels pour le criblage ou la sélection de souches de cellules transformées ainsi que des protéines nouvelles présentant un Intérêt en vue d'applications industrielles ou médicales.

L'un de ces modes opératoires résulte de l'idée de produire une série d'anticorps polyclonaux ou monoclonaux, obtenus de manière connue en soi, dirigée contre une protéine ou un autre type de molécule d'intérêt biochimique ou médical, cette molécule étant, ou pouvant être rendue, immunogénique, et d'utiliser ces anticorps comme sonde pour identifier parmi de très nombreux clones transformés par des gènes stochastiques ceux qui réagissent avec ces anticorps. Cette réaction résulte de l'homologie de structure existant entre le polypeptide synthétisé par le gène stochastique et la molécule initiale. On peut ainsi isoler de nombreuses protéines nouvelles se comportant comme des épitopes ou déterminants antigéniques de la molécule initiale. De telles protéines nouvelles sont susceptibles de simuler, stimuler, moduler ou bloquer l'effet de la molécule initiale. On comprendra que ce mode de sélection ou de criblage est, en lui-même, susceptible d'avoir de très nombreuses applications pharmacologiques et bio-médicales. On va maintenant décrite, à titre d'exemple non limitatif, ce premier mode opératoire en relation avec un cas particulier :

L'EGF (epidermal growth factor) est une petite protéine présente dans le sang et dont le rôle est de stimuler la croissance des cellules épithéliales. Cet effet est obtenu par l'interaction de l'EGF avec un récepteur spécifique situé dans la membrane des cellules épithéliales.

On prépare des anticorps dirigés contre l'EGF par injection chez l'animal d'EGF couplé à KLH (keyhole limpet hemocyanin) pour augmenter l'immunogénicité de l'EGF. Les anticorps anti-EGF des animaux immunisés sont purifiés, par exemple par passage sur une colonne d'affinité, dont le ligand est l'EGF ou un peptide de synthèse correspondant à un fragment d'EGF. Les anticorps anti-EGF purifiés sont utilisés comme sonde pour le criblage d'un grand nombre de clones bactériens lysés au chloroforme sur support solide. Les anticorps anti-EGF se combinent aux peptides ou protéines stochastiques dont les épitopes ressemblent à ceux de l'antigène initial. Les clones contenant ces peptides ou protéines sont mis en évidence par autoradiographie après incubation des supports solides avec de la protéine A radioactive ou après incubation avec un anticorps anti-anticorps radioactif.

Ces étapes identifient les clones contenant chacun une protéine (et son gène) réagissant avec l'anticorps de criblage. On peut ainsi faire des criblages parmi un très grand nombre de souches cellulaires bactériennes ou de plaques virales et il est possible de détecter des quantités extrêmement faibles, par exemple de l'ordre de 1 ng, de protéine produite. On procède alors à la culture des clones identifiés puis à la purification des protéines détectées par les moyens conventionnels. Les protéines purifiées sont testées in vitro dans des cultures de cellules épithéliales pour déterminer si elles inhibent, simulent ou modulent l'effet sur ces cultures de l'EGF. Certaines des protéines obtenues parce moyen sont susceptibles d'être utilisées pour le traitement chimiothérapique des épithéliomes. Les activités des protéines ainsi obtenues peuvent être améliorées par mutation de l'ADN codant pour les protéines, de manière analogue à celle décrite ci-dessus. Une variante de ce mode opératoire consiste à purifier des peptides, polypeptides ou protéines stochastiques, pouvant être utilisées comme vaccins ou plus généralement pouvant être utilisées pour conférer une immunité contre un agent pathogène ou pour exercer d'autres effets sur le système immunologique, par exemple, créer une tolérance ou diminuer l'hypersensibilité à l'égard d'un antigène donné, notamment par suite de la combinaison de ces peptides, polypeptides ou protéines avec les anticorps dirigés contre cet antigène. On comprendra que l'on peut utiliser ainsi ces peptides, polypeptides ou protéines aussi bien in vitro qu'in vivo.

Plus précisément, par exemple, dans l'ensemble des protéines nouvelles qui réagissent avec l'anticorps contre un antigène donné X, chacune a au moins un épitope en commun avec X, donc l'ensemble a un ensemble d'épitopes commun avec X. Ceci permet d'utiliser l'ensemble ou un sous-ensemble comme vaccin pour conférer l'immunité contre X. Il est, par exemple, aisé de purifier une ou plusieurs des protéines de capside du virus de l'hépatite B. Ces protéines sont injectées chez l'animal, par exemple le lapin, et on recueille les anticorps correspondant à l'antigène de départ par purification sur colonne d'affinité. On utilise ces anticorps, de la manière décrite ci-dessus, pour identifier les clones produisant une protéine ayant un épitope semblable à l'un au moins des épitopes de l'antigène initial. Après purification, on utilise ces protéines comme antigène (soit isolément, soit en combinaison) dans le but de conférer une protection contre l'hépatite B. La production ultérieure du vaccin ne nécessite plus le recours à l'agent pathogène initial.

Il est à remarquer que, lors de la description des modes opératoires qui précèdent, un certain nombres de manières de procéder à la sélection et au criblage ont été décrits. Tous ces modes opératoires impliquent la purification d'une protéine particulière à partir d'une souche transformée. Ces purifications de protéines peuvent être effectuées conformément aux méthodes usuelles et font appel, en particulier, aux techniques de chromatographie sur gel, sur échangeur d'ion, et à la chromatographie d'affinité. Par ailleurs, les protéines issues de gènes produits de manière stochastique peuvent avoir été clonées sous forme de protéines hybrides, comportant, par exemple, une séquence de l'enzyme β-galactosidase permettant la chromatographie d'affinités sur anticorps anti-β-galactosidase et permettant le clivage subséquent de la partie hybride (c'est-à-dire permettant de séparer la partie nouvelle de la partie bactérienne).On va maintenant décrire le principe et la mise en oeuvre de la sélection des peptides ou polypeptides et des gènes correspondants, exprimant ces peptides ou polypeptides, conformément à une deuxième méthode de criblage ou sélection fondée sur la détection de l'aptitude de ces peptides ou polypeptides à catalyser une réaction spécifique.

A titre d'exemple concret non limitatif, on va décrire la mise en oeuvre du criblage ou de la sélection dans 1e cas particulier de protéines capables de catalyser le clivage du lactose, ce qui est normalement une fonction remplie par l'enzyme β-galactosidase (β-gal).

Comme décrit ci-dessus, la première étape du procédé consiste à engendrer un très grand ensemble de vecteurs d'expression exprimant chacun une protéine nouvelle distincte. De manière concrète, on peut, par exemple, choisir le vecteur d'expression pUC8 avec clonage de séquences stochastiques d'ADN au site de restriction Pst 1.
Les plasmides ainsi obtenus sont ensuite introduits dans une souche de E. coli dans le génome de laquelle on a éliminé par les méthodes génétiques conventionnelles le gène naturel pour la β-galactosidase, Z, et un deuxième gène EBG, sans rapport avec le le premier, mais capable de muter vers la fonction β-gal. De telles cellules-hôtes (Z-, EBG-) ne sont pas capables par elles-mêmes de catalyser l'hydrolyse du lactose et, par conséquent, d'utiliser le lactose comme source de carbone pour la croissance. Ceci permet d'utiliser cette souche hôte pour le criblage ou la sélection de la fonction β-gal.

Une méthode d'essai biologique qui convient pour l'étude des souches transformées de E. coli présentant des gènes nouveaux exprimant la fonction β-gal consiste dans la culture des bactéries ainsi transformées dans des boîtes de Pétri renfermant le milieu X-gal. Dans ce cas, toute colonie bactérienne exprimant une fonction β-gal est visualisée sous forme d'une colonie bleue. En utilisant un tel essai biologique, on peut détecter même une activité catalytique faible. L'activité spécifique des enzymes les plus caractéristiques s'établit entre 10 et 10 000 molécules de produit par seconde.

En supposant qu'une protéine synthétisée par un gène stochastique présente une activité spécifique faible, de l'ordre de une molécule par 100 secondes, il serait toujours possible de détecter une telle activité catalytique. Dans une boîte de Pétri renfermant le milieu X-gal, en présence de l'inducteur non métabolisable IP-TG (isopropyl-D-thiogalactoside) la visualisation d'une région bleue requiert le clivage d'environ 10¹⁰ à 10¹¹ molécules de X-gal par millimètre carré. Une colonie bactérienne exprimant un enzyme faible et occupant une superficie de 1 mm² contient environ 10⁷ à 10⁸ cellules. Si chaque cellule présente une seule copie de l'enzyme faible, chaque cellule doit catalyser entre 10 000 et 100 clivages de X-gal afin de pouvoir être détectée, ce qui prend environ 2,7 à 270 heures. Etant donné que, dans des conditions sélectives, on peut s'attendre à une amplification du nombre des copies de plasmides par cellules, par exemple de 5 5 à 20 copies par cellule et même de 100 à 1000, et compte tenu du fait que jusqu'à 10% des protéines de la cellule peuvent être spécifiées par le nouveau gène, la durée nécessaire à la détection d'une colonie bleue dans le cas de 100 molécules d'enzymes de faible activité spécifique par cellule serait de l'ordre de 0,27 heure à 2,7 heures.

Parconséquent, le criblage d'un grand nombre de colonies bactériennes indépendantes, exprimant chacun un gène nouveau différent, en prenant comme critère de sélection la faculté d'exprimer la fonction β-gal est parfaitement réalisable. On peut ainsi réaliser le criblage d'environ 2000 colonies dans une boite de Pétri ordinaire ayant 10 cm de diamètre. Par conséquent, on peut cribler environ 20 millions de colonies sur une feuille d'agar X-gal de 1 m².

II est à remarquer que les colonies bactériennes apparaissant en bleu sur les boîtes de Pétri X-gal pourraient être faussement positives en raison d'une mutation dans le gènome bactérien lui conférant la capacité de métaboliser le lactose, ou pour d'autre raisons que celles qui résultent de l'activité catalytique de la protéine nouvelle exprimée par les cellules de la colonie. De tels faux positifs peuvent être directement éliminés en purifiant l'ADN des vecteurs d'expression provenant des colonies positives et en retransformant les cellules-hôtes E. Coli Z⁻,EBG⁻. Si l'activité β-gal résulte de la nouvelle protéine codée par le nouveau gène dans le vecteur d'expression, toutes les cellules transformées par ce vecteur devraient présenter la fonction β-gal. Au contraire, si la colonie bleue initiale résulte d'une mutation dans le génome de la cellule hôte, il s'agit d'un événement exceptionnel indépendant de la transformation et le nombre des cellules de la nouvelle souche transformée E. Coli servant d'hôte susceptible d'exprimer la fonction β-gal devrait être petit ou nul.

On insistera particulièrement sur la puissance de la purification en masse simultanée des vecteurs d'expression pour tous les clones positifs (bleus) suivie par la retransformation des bactéries naïves. Supposons que l'on désire effectuer un criblage en vue de sélectionner des protéines ayant une fonction catalytique et que la probabilité qu'un nouveau peptide ou polypeptide remplisse cette fonction, au moins faiblement, soit de 10⁻⁶, alors que la probabilité que la souche microbienne hôte E. Coli est sujette à une mutation ayant pour effet qu'elle devienne capable de remplir cette fonction, soit de 10⁻⁵ , on peut calculer que sur20 millions de bactéries transformées soumises au criblage, 20 clones positifs seront, en moyenne, attribuables aux gènes nouveaux sur les vecteurs d'expression que chacune comporte, alors que 200 clones positifs résulteront de mutations d'arrière-plan. La purification en masse des vecteurs d'expression de l'intégralité des 220 clones bactériens positifs et la retransformation des bactéries naïves avec les vecteurs d'expression mis en commun produira un grand nombre de clones positifs constitués de toutes les bactéries transformées avec les 20 vecteurs d'expression qui codent les nouvelles protéines ayant la fonction désirée, et un très petit nombre de clones bactériens résultant de mutations d'arrière-plan contenant les 200 vecteurs d'expression restant sans intérêt. Un petit nombre de cycles de purification de vecteurs d'expression dans les colonies bactériennes positives, ainsi que de retransformation, permet la détection des très rares vecteurs d'expression véritablement positifs par rapport à une activité catalytique désirée, malgré un bruit de fond très élevé de mutations des cellules-hôtes pour cette fonction.

A la suite d'opérations de criblage de ce genre, on peut purifier la nouvelle protéine grâce à la mise en oeuvre de techniques usuelles. La production de cette protéine en grande quantité est rendue possible grâce au fait que l'identification de la protéine utile s'accompagne de l'identification simultanée du gène codant cette protéine.
Par conséquent, on peut utiliser le vecteur d'expression lui-même ou bien on peut transplanter le gène nouveau dans un vecteur d'expression plus approprié à la synthèse et à l'isolation en grande quantité.
On peut appliquer des modes de criblage de ce genre à toute fonction enzymatique pour laquelle il existe un essai biologique approprié. De tels criblages ne nécessitent pas que la fonction enzymatique que l'on recherche soit profitable à la cellule hôte. On peut effectuer un criblage non seulement par rapport à une fonction enzymatique mais également pour toute autre propriété désirée pour laquelle on peut établir un essai biologique approprié. On peut ainsi effectuer même dans le cas simple de la fonction β-gal visualisée sur boîte de Pétri à milieu X-gal, le criblage d'un nombre de gènes nouveaux de l'ordre de 100 millions ou même d'un milliard pour une activité catalytique ou une autre propriété désirée.

### Sélection des cellules-hôtes modifiées

D'autre part, on peut utiliser des techniques de sélection pourtoute propriété, catalytique ou autre, dont la présence ou l'absence peut être rendue essentielle à la survie des cellules-hôtes contenant les vecteurs d'expression codant les gènes nouveaux ou pouvant être utiles pour la sélection de virus codant et exprimant le gène nouveau. A titre d'exemple concret, non limitatif, on va maintenant revenir sur la description du mode de sélection en relation avec la fonction β-galactosidase. Une souche appropriée Z-EBG- d'E. Coli ne peut pas croître en utilisant le lactose comme seule source de carbone. Ainsi, après la mise en oeuvre de la première étape décrite ci-dessus, on peut cultiver un très grand nombre d'hôtes transformés par des vecteurs d'expression codant les gènes nouveaux, la culture étant effectuée dans des conditions sélectives, soit en diminuant progressivement la concentration des autres sources de carbone, soit en utilisant seulement le lactose dès le départ. Au cours d'une telle sélection. la mutagénèse in vivo par voie de recombinaison ou par récupération explicite de vecteurs d'expression et mutagénèse in vitro de leurs gènes nouveaux par des mutagènes variés, ou par tout autre technique usuelle, permet des améliorations adaptatives de l'aptitude à remplir la fonction catalytique désirée. Lorsqu'il existe à la foi des techniques de sélection et des techniques commodes d'essai biologique, comme dans le cas du présent exemple, on peut utiliser initialement des techniques de sélection pour enrichir la représentation en bactéries hôte exprimant la fonction β-gal, puis effectuer un criblage sur boite de Pétri à milieu X-gal afin d'identifier efficacement des cellules positives. En l'absence de technique d'essai biologique convenable, l'application de conditions de sélection de plus en plus rigoureuses constitue la voie la plus facile pour purifier un type ou un petit nombre de types de cellules-hôtes distinctes dont les vecteurs d'expression codent les protéines catalysant la réaction choisie.

On peut utiliser ces techniques pour trouver de nouvelles protéines ayant une grande variété de caractéristiques structurelles ou fonctionnelles en plus de l'aptitude à catalyser des réactions spécifiques. Par exemple, on peut effectuer un criblage ou une sélection pour trouver de nouvelles protéines se fixant sur des sites cis-régulateurs de l'ADN et bloquant de ce fait l'expression d'une fonction des cellules hôtes, ou encore bloquant latranscription de l'ADN, stimulant la transcription, etc.

Par exemple, dans le cas de E. Coli une souche mutante du répresseur de l'opéron lactose (i-) exprime de manière constitutive la fonction β-gal en raison du fait que l'opérateur lactose n'est pas réprimé. Toutes les cellules de ce genre produisent des clones bleus sur les boîtes de Pétri contenant le milieu X-gal. Il est possible de transformer de telles souches hôtes avec des vecteurs d'expression synthétisant des protéines nouvelles et effectuer un criblage sur boîte de Pétri à milieu X-gal en vue de détecter les clones qui ne sont pas bleus. Parmi ceux-ci, certains représentent des cas où la nouvelle protéine se fixe sur l'opérateur lactose et réprime la synthèse de β-gal. On peut purifier en masse de tels plasmides, les retransformer, isoler les clones qui ne produisent pas la β-gal et effectuer ensuite une vérification détaillée.

Comme il est mentionné plus haut, le procédé peut être utilisé aux fins de créer puis d'isoler non seulement des protéines exploitables mais aussi des ARN et des ADN constituant des produits en soi, dotés de propriétés exploitables. Ceci résulte évidemment du fait que d'une part, le procédé consiste à créer des séquences stochastiques d'ADN, susceptibles d'interagir directement avec d'autres constituants cellulaires ou biochimiques, et que d'autre part ces séquences clonées dans un vecteur d'expression sont transcrites en ARN eux aussi capables de multiples interactions biochimiques.

### Exemple de mise en oeuvre du procédé pour la création et la sélection d'un ADN exploitable en soi:

Cet exemple illustre la sélection d'un ADN exploitable et le purification et l'étude du mécanisme d'action des protéines régulatrices se liant à l'ADN. Soit une préparation du récepteur de l'oestradiol, une protéine obtenue par une méthode connue en soi. En présence d'oestradiol, une hormone stéroïde sexuelle, ce récepteur change de conformation et se lie fortement à certaines séquences spécifiques de l'ADN génomique, affectant ainsi la transcription de gènes impliqués dans la différenciation sexuelle et le contrôle de la fertilité.
En incubant une mélange constitué d'oestradiol, de son récepteur et d'un grand nombre de séquences stochastiques différant insérées dans leur vecteur puis en filtrant le mélange à travers une membrane de nitro cellulose on obtient une sélection directe des séquences stochastiques se liant au complexe oestrogène - récepteur puisque seuls les ADN liés à une protéine sont retenus par la membrane. Après lavage et élution, l'ADN libéré de la membrane est utilisé tel quel pour transformer des bactéries. Après culture des bactéries transformées, on purifie à nouveau les vecteurs qu'elles contiennent et on procède à un ou plusieurs cycles d'incubation, filtration et transformation comme décrit ci-dessus. Ces opérations permettent d'isoler des séquences stochastiques d'ADN dotées d'une affinité élevée pour le complexe oestradiol-récepteur. De telles séquences sont susceptibles de nombreuses applications diagnostiques et pharmacologiques, en particulier pour la mise au point d'oestrogènes de synthèse pour le contrôle de là fertilité et le traitement de la stérilité.

### Création et sélection d'un ARN exploitable en soi

Soit un grand nombre de séquences d'ADN stochastique produites comme il a été décrit et clonées dans un vecteur d'expression. II va de soi que l'ARN transcrit à partir de ces séquences dans des cellules-hôtes transformées peut être un produit exploitable en soi.
A titre d'exemple non limitatif, on peut sélectionner un gène stochastique codant pour un ARN de transfert (t-ARN) suppresseur par la procédure suivante :

On transforme par un grand nombre (≥ 10⁸) de séquences stochastiques, une souche bactérienne compétente comportant une mutation "nonsense" dans le gène arg E. On étale les bactéries transformées sur milieu minimal sans arginine et contenant l'antibiotique de sélection du plasmide (ampicilline s'il s'agit du vecteur pUC8).Seules les bactéries transformées qui sont devenues capables de synthétiser l'arginine pourront croître. Ce phénotype peut résulter soit d'une mutation en retour soit de l'introduction dans la cellule d'un suppresseur. Il est facile de tester chaque colonie transformée pour déterminer si le phénotype arg⁺ résulte ou non de la présence du gène produit de manière stochastique dans son vecteur : on se contente de préparer le plasmide de cette colonie et de vérifier qu'il confère le phénotype Arg⁺ à toute cellule arg E qu'il transforme.

### Sélection de protéines capables de catalyser une séquence de réaction

On va maintenant décrire un autre mode de sélection, qui est susceptible d'applications indépendantes, fondé sur le principe de la sélection simultanée, en parallèle, d'un certain nombre de nouvelles protéines capables de catalyser une séquence de réactions connectées entre elles.

L'idée mère de cette méthode est la suivante : étant donné un ensemble de composés chimiques de départ considérés comme des "briques" ou des éléments de construction à partir desquels on désire effectuer la synthèse d'un ou de plusieurs composés chimiques désirés par l'intermédiaire d'une séquence de réactions chimiques catalysées, il existe un très grand nombre de voies de réaction pouvant se substituer les unes aux autres complètement ou en partie, qui sont toutes possibles du point de vue thermodynamique et qui conduisent de l'ensemble des "briques" ou blocs de construction au composé chimique cible désiré. Une synthèse efficace d'un composé cible est favorisée si chaque étape d'au moins l'un des trajets réactionnels menant de l'ensemble des "blocs de construction" au composé cible est constitué de réactions qui sont chacune catalysées. Toutefois, il est relativement moins important de déterminer parmi les nombreux chemins réactionnels complètement ou partiellement indépendants ceux qui bénéficient de la catalyse, Dans la description qui précède, on a montré comment il était possible d'obtenir un très grand nombre de cellules hôtes qui expriment chacune une protéine nouvelle distincte. Chacune de ces protéines nouvelles est susceptible de catalyser l'une quelconque des réactions possibles dans l'ensemble de toutes les réactions possibles conduisant de l'ensemble des blocs de construction au composé cible. Si un nombre de protéines stochastiques suffisamment grand est présent dans un mélange réactionnel contenant les composés constituant les blocs de construction, de sorte qu'un nombre suffisamment élevé des réactions possibles se trouve catalysé, il y a une forte probabilité qu'une séquence de réactions connectées entre elles pour conduire de l'ensemble de blocs de construction au composé cible sera catalysée par un sous-ensemble des nouvelles protéines. Il est évident que le procédé peut s'étendre à la catalyse non seulement d'un, mais de plusieurs composés cibles, simultanément.

En se fondant sur le principe qui vient d'être énoncé, on peut procéder de la manière suivante pour sélectionner en parallèle un ensemble de nouvelles protéines catalysant une séquence désirée de réactions chimiques :
1. Spécifier l'ensemble désiré de composés constituant les "blocs de construction" en utilisant de préférence un nombre raisonnablement élevé d'espèces chimiques distinctes afin d'augmenter le nombre de voies potentielles concurrentes menant au composé chimique cible désiré.
2. Dans un volume approprié d'une solution de milieu réactionnel, ajouter un nombre très grand de nouvelles protéines stochastiques isolées à partir des cellules transformées ou transfectées synthétisant ces protéines. Effectuer un essai pour déterminer si le composé cible est formé. Si c'est le cas, confirmer que cette formation nécessite la présence du mélange des protéines nouvelles, Si c'est le cas, ce mélange doit contenir un sous-ensemble de protéines catalysant une ou plusieurs séquences de réaction menant de l'ensemble de "blocs de construction" au composé cible. Purifier en divisant la réserve initiale de clones qui synthétisent l'ensemble de nouvelles protéines. stochastiques, le sous-ensemble nécessaire pour catalyser la séquence de réactions conduisant au produit cible.

Plus précisément, à titre d'exemple non limitatif, on va décrire la sélection d'un ensemble de nouvelles protéines aptes à catalyser la synthèse d'un petit peptide spécifié, à savoir un pentapeptide, à partir d'un ensemble de blocs de construction constitué de peptides plus petits et d'acides aminés. Tout peptide est constitué par une séquence linéaire de 20 types différents d'acides aminés, orientée de son extrémité amino à son extrémité carboxe. Tout peptide peut être formé en une étape par condensation terminale de deux peptides plus petits (ou de deux acides aminés), ou par hydrolyse d'un peptide plus grand. Un peptide comprenant M résidus pourra donc être formé d'un nombre égal à M -1 réactions de condensation. Le nombre de réactions, R, par lequel un ensemble de peptides ayant une longueur de 1, 2, 3... M résidus, peut être interconverti est plus grand que le nombre d'espèces moléculaires T. Ceci s'exprime R/_{T} ≃ M-2. Ainsi, en partant d'un ensemble donné de peptides, un grand nombre de chemins réactionnels indépendants ou partiellement indépendants mène à la synthèse d'un pentapeptide cible spécifique. On peut choisir un pentapeptide dont la présence peut être commodément décelée grâce à un essai effectué par des techniques usuelles, par exemple par analyse HPLC (chromatographie en phase liquide sous haute pression), chromatographie sur papier, etc. La formation de la liaison peptidique requiert de l'énergie en milieu aqueux dilué mais, si les peptides participant aux réactions de condensation sont suffisamment concentrés, c'est la formation de la liaison peptidique plutôt que l'hydrolyse qui se trouve thermodynamiquement favorisée et qui se produit avec un rendement élevé en présence d'un catalyseur enzymatique approprié, par exemple la pepsine ou la trypsine, sans nécessiter la présence d'ATP ou d'autres composés à haute énergie. On peut utiliser un tel mélange réactionnel de peptides de petite taille et dont les acides aminés sont marqués radioactivement, au moyen de traceurs radioactifs du type ³H, ¹⁴C, ³⁵S, pour constituer l'ensemble de blocs de construction à concentration suffisamment élevée pour conduire aux réactions de condensation.

On peut, par exemple, procéder de la manière suivante :
on dissout environ 15mg de chaque acide aminé et petit peptide ayant 2 à 4 acides aminés, choisis pour constituer l'ensemble de blocs de construction, dans un volume de 0,25 ml à 1,0 ml de tampon phosphate à 0,1 M, pH 7,6. On purifie un grand nombre de protéines nouvelles engendrées et isolées comme décrit ci-dessus à partir de leur hôte bactérien ou autre. On dissout le mélange de ces nouvelles protéines jusqu'à une concentration totale finale de l'ordre de 0,8 à 1,0 mg/ml, dans le même tampon. On ajoute 0,25 ml à 0,5 ml du mélange de protéines au mélange de blocs de construction. On incube à une température de 25°C à 40°C pendant 1 à 40 heures. On enlève des parties aliquotes de 8 ul à intervalles réguliers, dont le premier compte comme "témoin à blanc", avant l'adjonction du mélange de nouvelles protéines. On soumet les échantillons à une analyse par chromatographie en utilisant comme solvant un mélange de n-butanol-acide acétique-pyridine-eau (30:6: 20:24 en volume). On sèche le chromatogramme et on le révèle à la ninhydrine ou on l'autoradiographie (avec ou sans écran d'intensification). Du fait que les composés constituant les éléments de construction sont marqués radioactivement, le composé cible sera radioactif et il aura une activité spécifique élevée permettant sa détection au niveau de 1 à 10 ng. Au lieu de l'analyse par chromatographie habituelle, on peut effectuer une analyse par HPLC (high pressure liquid chromatography) qui est plus rapide et plus simple à effectuer. De manière générale, on peut utiliser les procédés d'analyses usuels. De la sorte, on peut détecter un rendement en composés cibles inférieur à une partie par million en poids par rapport aux composés ou "matériaux de construction" initiaux.

Dans le cas où le pentapeptide est formé dans les conditions décrites ci-dessus mais où sa formation n'a pas lieu lorsqu'on utilise un extrait purifié comme ci dessus mais obtenu à partir de cellules transformées par le vecteur d'expression dépourvu d'insertion stochastique, la formation du pentapeptide ne résulte pas de la présence des contaminants bactériens et requiert donc la présence d'un sous-ensemble de nouvelles protéines dans le mélange réactionnel.

L'étape suivante consiste dans la séparation du sous-ensemble particulier de cellules qui contiennent les vecteurs d'expression des nouvelles protéines catalysant la séquence de réactions conduisant au pentapeptide cible. Par exemple si le nombre de réactions formant cette séquences est égal à 5, il y a environ 5 protéines nouvelles qui catalysent les réactions nécessaire. Dans le cas où la "banque de clones" de bactéries contenant les vecteurs d'expression codant les gènes nouveaux contient un nombre de gènes nouveaux distincts de l'ordre de 1 000 000 , on effectue l'isolation en masse de tous ces vecteurs d'expression et la retransformation de 100 ensembles distincts de 10⁸ bactéries avec un rapport de vecteurs à bactéries suffisamment faible pour que, en moyenne chaque ensemble de bactéries soit transformé par seulement environ la moitié du nombre de gène initiaux, c'est-à-dire environ 500 000. Par conséquent,la probabilité pour qu'un quelconque de ces 100 ensembles de bactéries contienne l'ensemble des 5 nouvelles protéines critiques est égal à (1/2)⁵=1/32. Parmi les 100 ensembles initiaux de bactéries; environ 3 contiendront les 5 transformants critiques. Dans chacun de ces ensembles, la quantité totale de gènes nouveaux présente n'est plus que de 500 000 au lieu de un million. Par répétitions successives, dont le nombre total est dans le cas présent de 20, de cette procédure on peut isoler les 5 nouveaux gènes critiques. Après quoi, la mutagénèse et la sélection de cet ensemble de 5 gènes stochastique permet la recherche de fonctions catalytiques améliorées. Dans le cas où il est nécessaire de catalyser une séquence de réactions comprenant un nombre de réactions de l'ordre de 20 et que 20 gènes codant des nouvelles protéines doivent être isolés en parallèle, il suffit d'ajuster la multiplicité des transformations de sorte que chaque ensemble de 10⁸ bactéries reçoivent 80% ces 10⁶ gènes stochastiques en utilisant 200 ensembles de ce genre. La probabilité que les 20 nouvelles protéines se trouvent dans un ensemble donné de bactéries est de 0,8²⁰ c'est-à-dire environ 0,015. Par conséquent, 2 parmi les 200 ensembles contiendront les 20 nouveaux gènes nécessaires pour catalyser la formation du composé cible. Le nombre de répétitions de cycles nécessaires pour l'isolation des 20 nouveaux gènes est de l'ordre de 30.

Les principes et les modes opératoires énoncés ci-dessus se généralisent du cas des peptides à de nombreux domaines de la chimie dans lesquels on peut effectuer des réactions en milieu aqueux dans des conditions de pH, température et concentration des solutions permettant les fonctions enzymatiques générales. Dans chaque cas, il est nécessaire de pouvoir disposer d'une méthode d'essai pour déceler la formation du ou des composés cibles désirés. II faut aussi choisir un nombre suffisamment élevé de "blocs de construction" pour augmenter le nombre de séquences de réaction qui conduisent au composé cible.

L'ensemble concret qui vient d'être donné de la synthèse d'un pentapeptide cible peut être généralisé de la manière suivante :

Le procédé tel qu'il est décrit, engendre, entre autres produits, des peptides et protéines produits de manière stochastique. Ces peptides ou protéines peuvent agir, par voie catalytique ou autre, sur d'autres composés. Ils peuvent également constituer les substrats sur lesquels ils agissent. On peut ainsi sélectionner (ou cribler) la capacité qu'ont les peptides ou protéines stochastiques d'interagir entre eux et de ce fait de modifier la conformation, la structure ou la fonction de certains d'entre eux. De même, on peut sélectionner (ou cribler) la capacité de ces peptides et protéines à catalyser entre eux l'hydrolyse, la condensation, le transpeptidation ou d'autres réactions de modification. Par exemple, l'hydrolyse d'une protéine produit de manière stochastique donnée par un membre au moins de l'ensemble des peptides et protéines stochastiques peut être suivie et mesurée par marquage radioactif de la protéine donnée suivi d'une incubation avec le mélange des protéines produits de manière stochastique, en présence d'ions tels que Mg, Ca, Zn, Fe et des composés ATP et GTP. On mesure ensuite l'apparition des fragments radioactifs de la protéine marquée, comme il a été décrit. La ou les protéines produites de manière stochastique qui catalysent cette réaction peuvent alors être isolées, ainsi que leur gène producteur, par diminution séquentielle de la librairie de clones transformants, comme décrit.

Une extension du procédé consiste en la sélection d'un ensemble de peptides et polypeptides produits de manière stochastique capables de catalyser une suite de réactions menant des constituant de départ (acides aminés et petits peptides) à certains des peptides ou polypeptides de l'ensemble. Il est ainsi envisageable de sélectionner un ensemble capable de catalyser sa propre synthèse : un tel ensemble réflexivement autocatalytique peut s'établir dans un chemostat où les produits de réaction sont constamment dilués mais où la concentration des produits de départ est maintenues constante. On peut vérifier l'existence d'un ensemble de cette nature par chromatographie sur gel à deux dimensions et par "HPLC" montrant la synthèse d'une distribution stable de peptides et de polypeptides. Les volumes de réaction dépendent du nombre d'espèces moléculaires utilisées et des concentrations nécessaires pour favoriser la formation de liaisons peptidiques par rapport à l'hydrolyse. La distribution des espèces moléculaires d'un ensemble autocatalytique est susceptible de varier ou de dériver par suite de l'émergence d'ensembles autocatalytiques variants. Les peptides et polypeptides qui constituent un ensemble autocatalytique peuvent avoir certains éléments en commun avec le vaste ensemble de départ (constitué des peptides et polypeptides codés selon le procédé) mais peuvent aussi contenir des peptides et polypeptides non codés par l'ensemble des gènes produits de manière stochastique codant l'ensemble de départ.

L'ensemble des gènes produits de manière stochastique dont les produits sont nécessaire pour établir un tel ensemble autocatalytique peut être isolé comme il a été décrit, par diminutions séquentielles de la libraire de clones transformants. Par ailleurs, un ensemble autocatalytique peut contenir des peptides codés initialement par les gènes produits de manière stochastique et formés de manière continue dans l'ensemble autocatalytique. Pour isoler ce sous-ensemble codé de peptides et de protéines on peut utiliser l'ensemble autocatalytique pour obtenir, par immunisation chez l'animal, des sérums polyclonaux reconnaissant un très grand nombre des constituants de l'ensemble autocatalytique.

Ces sérums peuvent ensuite être utilisés pour cribler la librairie de gènes produits de manière stochastique pour y trouver les gènes exprimant des protéines capables de se combiner aux anticorps présents dans les sérums.

Cet ensemble de gènes produits de manière stochastique exprime un grand nombre des protéines produites de manière stochastique codées qui persistent dans l'ensemble autocatalytique. Le reste des constituants codés d'un tel ensemble autocatalytique peut être isolé par diminution séquentielle, comme décrit, de la librairie de gènes produits de manière stochastique dont le sous-ensemble détecté par la méthode immunologique a été soustrait.

Les ensembles autocatalytiques de peptides et de protéines obtenue de la manière qui vient d'être décrite sont susceptibles de trouver de nombreuses applications pratiques.

## Revendications

1. Méthode d'identification d'un peptide, d'un polypeptide ou d'une protéine qui se lie à un ligand, comprenant :
(a) la production d'une population de peptides, de polypeptides ou de protéines encodés par une population d'au moins 1x10 ⁸ différentes séquences de polynucléotides synthetisés de manière stochastique, et
(b) l'examen de ladite population de peptides, de polypeptides ou de protéines en vue de ladite liaison audit ligand dans des conditions qui permettent la détection d'un ou de plusieurs peptides, polypeptides ou d'une ou de plusieurs protéines liés audit ligand.

2. Méthode selon la revendication 1, **caractérisée en ce que** l'étape (a) comprend en outre la synthèse d'une population de séquences de polynucléotides produites de manière stochastique et la traduction de ladite population de séquences de polynucléotides pour produire ladite population de peptides, de polypeptides ou de protéines.

3. Méthode selon la revendication 1, **caractérisée en ce que** l'étape (a) comprend en outre la synthèse d'une population de séquences de polynucléotides au moins partiellement stochastiques et produites de manière stochastique, et la traduction de ladite population de séquences de polynucléotides pour produire ladite population de peptides; de polypeptides ou de protéines.

4. Méthode selon la revendication 2 ou la revendication 3, comprenant en outre l'amplifïcation de ladite population de séquences de polynucléotides produites de manière stochastique.

5. Méthode selon la revendication 1, **caractérisée en ce que** l'étape (a) comprend en outre la synthèse d'une population de séquences de polynucléotides produites de manière stochastique et l'expression de ladite population de séquences de polynucléotides pour produire ladite population de peptides, de polypeptides ou de protéines.

6. Méthode selon la revendication 1, **caractérisée en ce que** l'étape (a) comprend en outre la synthèse d'une population de séquences de polynucléotides au moins partiellement stochastiques et produites de manière stochastique et l'expression de ladite population de séquences de polynucléotides pour produire ladite population de peptides, de polypeptides ou de protéines.

7. Méthode selon la revendication 1, comprenant en outre l'isolément de la séquence de polynucléotides encodant un ou plusieurs desdits peptides, polypeptides ou protéines qui se lient audit ligand.

8. Méthode selon la revendication 1, comprenant en outre l'amélioration de ladite liaison audit ligand par mutagénèse *in vitro* ou *in vivo.*

9. Méthode de production d'un peptide, d'un polypeptide ou d'une protéine qui se lie à un ligand, comprenant :
(a) la production d'une population de peptides, de polypeptides ou de protéines encodés par une population d'au moins 1×10⁸ séquences de polynucléotides synthetisés de manière stochastique,
(b) l'examen de ladite population de peptides, de polypeptides ou de protéines en vue de ladite liaison audit ligand dans des conditions qui permettent la détection d' un ou de plusieurs peptides, polypeptides ou d'une ou de plusieurs protéines liés audit ligand ;
(c) l'isolement de la séquence de polynucléotides encodant un ou plusieurs desdits peptides, polypeptides ou protéines qui se lient audit ligand, et
(d) la production dudit peptide, dudit polypeptide ou de ladite protéine.

10. Méthode d'isolement d'une séquence de polynucléotides encodant un peptide, un polypeptide ou une protéine qui se lie à un ligand, comprenant :
(a) la production d'une population de peptides, de polypeptides ou de protéines encodés par une population d'au moins 1×10⁸ séquences de polynucléotides synthetisés de manière stochastique,
(b) l'examen de ladite population de peptides, de polypeptides ou de protéines en vue de ladite liaison audit ligand dans des conditions qui permettent la détection d'un ou de plusieurs peptides, polypeptides ou d'une ou de plusieurs protéines liés audit ligand, et
(c) l'isolement de la séquence ou des séquences de polynucléotides encodant ledit peptide, ledit polypeptide ou ladite protéine qui se lient à un ligand.

11. Méthode de production d'une population diverse de cellules hôtes comprenant :
(a) la synthèse d'une population diverse de séquences de polynucléotides produites de manière stochastique, ladite population comprenant au moins 1×10 ⁸ différentes séquences de polynucléotides ;
(b) l'insertion de ladite population diverse de séquences de polynucléotides produites de manière stochastique dans une population de vecteurs pour former une population diverse de vecteurs contenant des séquences de polynucléotides produites de manière stochastique, et
(c) l'introduction de ladite population diverse de vecteurs dans des cellules hôtes.

12. Méthode d'identification d'un polynucléotide qui se lie à un ligand, comprenant :
(a) la production d'une population de polynucléotides comprenant au moins 1×10⁸ différentes séquences de polynucléotides synthetisés de manière stochastique, et
(b) l'examen de ladite population de polynucléotides en vue de ladite-liaison audit ligand dans des conditions qui permettent la détection d'un ou de plusieurs polynucléotides qui se lient audit ligand.

13. Méthode selon la revendication 12, comprenant en outre l'amplification de ladite population de séquences de polynucléotides produites de manière stochastique.

14. Méthode selon la revendication 12, **caractérisée en ce que** ladite population de polynucléotides comprend l'ADN.

15. Méthode selon la revendication 12, **caractérisée en ce que** ladite population de polynucléotides comprend l'ARN.

16. Méthode selon la revendication 12, comprenant en outre l'isolement d'une ou de plusieurs desdites séquences de polynucléotides qui se lient audit ligand.

17. Méthode selon la revendication 12, comprenant en outre l'amélioration de ladite propriété prédéterminée par mutagénèse *in vitro* ou *in vivo.*

18. Méthode de modification de la transcription ou de la réplication d'une séquence d'ADN, comprenant :
(a) la production d'une population de peptides, de polypeptides ou de protéines, encodés par des séquences de polynucléotides synthetisés de manière stochastique,
(b) l'inspection de ladite population de peptides, de polypeptides ou de protéines dans des conditions qui permettent l'identification d'un ou de plusieurs peptides, polypeptides ou d'une ou plusieurs protéines, qui modifient la transcription, ou la réplication d'une séquence d'ADN, et
(c) la mise en contact d'une séquence d'ADN avec un ou plusieurs desdits peptides, polypeptides ou protéines identifiés qui modifient la transcription ou la réplication d'une séquence d'ADN.

19. Méthode de production d'un peptide, d'un polypeptide, ou d'une protéine utile à la préparation d'un vaccin, comprenant les étapes :
(a) de production, par couplage enzymatique, de séquences de polynucléotides produites de manière stochastique ;
(b) de formation d'une bibliothèque de vecteurs d'expression contenant de telles séquences de polynucléotides produites de manière stochastique ;
(c) de la mise en culture de cellules hôtes contenant les vecteurs pour produire des peptides, des polypeptides, ou des protéines encodés par les séquences de polynucléotides produites de manière stochastique ;
(d) d'exécution de l'examen ou de la sélection de cellules hôtes de ce genre, pour identifier un peptide produit, un polypeptide produit, ou une protéine produite par les cellules hôtes ayant au moins un épitope similaire à une séquence d'acide aminés de l'un des épitopes d'un antigène doriné ;
(e) d'isolement d'une séquence de polynucléotides produite de manière stochastique qui encode le peptide, le polypeptide ou la protéine identifié(e), et
(f) d'utilisation de la séquence isolée pour produire le peptide, le polypeptide, ou la protéine ayant la propriété.

## Claims

1. A method of identifying a peptide, a polypeptide or a protein which binds to a ligand, comprising:
(a) the production of a population of peptides, polypeptides or proteins encoded by a population of at least 1x10⁸ different sequences of polynucleotides synthesised stochastically, and
(b) the examination of said population of peptides, polypeptides or proteins with a view to said binding to said ligand under conditions which permit detection of one or more peptides, polypeptides or one or more proteins bound to said ligand.

2. A method according to claim 1 **characterized in that** step (a) further comprises the synthesis of a population of sequences of polynucleotides produced stochastically and the translation of said population of sequences of polynucleotides to produce said population of peptides, polypeptides or proteins.

3. A method according to claim 1 **characterized in that** step (a) further comprises the synthesis of a population of sequences of polynucleotides which are at least partially stochastic and produced stochastically, and the translation of said population of sequences of polynucleotides to produce said population of peptides, polypeptides or proteins.

4. A method according to claim 2 or claim 3 and further comprising amplification of said population of sequences of polynucleotides produced stochastically.

5. A method according to claim 1 **characterized in that** step (a) further comprises the synthesis of a population of sequences of polynucleotides produced stochastically and the expression of said population sequences of polynucleotides to produce said population of peptides, polypeptides or proteins.

6. A method according to claim 1 **characterized in that** step (a) further comprises the synthesis of a population of sequences of polynucleotides which are at least partially stochastic and produced stochastically and the expression of said population of sequences of polynucleotides to produce said population of peptides, polypeptides or proteins.

7. A method according to claim 1 further comprising the isolation of the sequence of polynucleotides encoding one or more of said peptides, polypeptides or proteins which bind to said ligand.

8. A method according to claim 1 further comprising the improvement of said binding to said ligand by mutagenesis *in vitro* or *in vivo.*

9. A method of producing a peptide, a polypeptide or a protein which binds to a ligand, comprising:
(a) the production of a population of peptides, polypeptides or proteins encoded by a population of at least 1 x 10⁸ sequences of polynucleotides synthesised stochastically;
(b) the examination of said population of peptides, polypeptides or proteins with a view to said binding to said ligand under conditions which permit detection of one or more peptides, polypeptides or one or more proteins bound to said ligand,
(c) the isolation of the sequence of polynucleotides encoding one or more of said peptides, polypeptides or proteins which bind to said ligand, and
(d) the production of said peptide, said polypeptide, or said protein.

10. A method of isolating a sequence of polynucleotides encoding a peptide, a polypeptide or a protein which binds to a ligand, comprising:
(a) the production of a population of peptides, polypeptides or proteins encoded by a population of at least 1 x 10⁸ sequences of polynucleotides synthesised stochastically;
(b) the examination of said population of peptides, polypeptides or proteins with a view to said binding to said ligand under conditions which permit detection of one or more peptides, polypeptides or one or more proteins bound to said ligand,
(c) the isolation of the sequence or sequences of polynucleotides encoding said peptide, said polypeptide or said protein which bind to a ligand.

11. A method of producing a diverse population of host cells, comprising:
(a) the synthesis of a diverse population of sequences of polynucleotides synthesised stochastically, said population comprising at least 1 x 10⁸ different sequences of polynucleotides,
(b) the insertion of said diverse population of sequences of polynucleotides produced stochastically into a population of vectors to form a diverse population of vectors containing sequences of polynucleotides produced stochastically, and
(c) the introduction of said diverse population of vectors into host cells.

12. A method of identifying a polynucleotide which binds to a ligand, comprising:
(a) the production of a population of polynucleotides comprising at least 1 x 10⁸ different sequences of polynucleotides synthesised stochastically, and
(b) the examination of said population of polynucleotides with a view to said binding to said ligand under conditions which permit detection of one or more polynucleotides which bind to said ligand.

13. A method according to claim 12 further comprising the amplification of said population of sequences of polynucleotides produced stochastically.

14. A method according to claim 12 **characterized in that** said population of polynucleotides comprises DNA.

15. A method according to claim 12 **characterized in that** said population of polynucleotides comprises RNA.

16. A method according to claim 12 further comprising the isolation of one or more of said sequences of polynucleotides which bind to said ligand.

17. A method according to claim 12 further comprising the improvement of said predetermined property by mutagenesis *in vitro* or *in vivo.*

18. A method of modifying transcription or replication of a sequence of DNA, comprising:
(a) the production of a population of peptides, polypeptides or proteins encoded by sequences of polynucleotides synthesised stochastically,
(b) the inspection of said population of peptides, polypeptides, or proteins under conditions which permit the identification of one or more peptides, polypeptides or one or more proteins, which modify transcription, replication or stability of a sequence of DNA, and
(c) contacting a sequence of DNA with one or more of said peptides, polypeptides or proteins identified which modify the transcription or replication of a sequence of DNA.

19. A method of producing a peptide, a polypeptide or a protein which is useful for the preparation of a vaccine, comprising the steps:
(a) production by enzymatic coupling of sequence of polynucleotides produced stochastically,
(b) formation of a library of expression vectors containing such sequences of polynucleotides produced stochastically,
(c) culturing of host cells containing the vectors to produce peptides, polypeptides or proteins encoded by sequences of polynucleotides produced stochastically,
(d) execution of examination or selection of host cells of that kind to identify a peptide produced, a polypeptide produced, or a protein produced, by the host cells having at least one epitope similar to a sequence of amino acids of one of the epitopes of a given antigen,
(e) isolation of a sequence of polynucleotides produced stochastically, which encodes the peptide, the polypeptide or the protein identified, and
(f) use of the isolated sequence to produce the peptide, the polypeptide or the protein having the property.

## Patentansprüche

1. Verfahren zum Identifizieren eines Peptids, eines Polypeptids oder eines Proteins, das an einen Liganden bindet, aufweisend:
(a) Die Herstellung eines Bestands an Peptiden, an Polypeptiden oder an Proteinen, die codiert werden von einem Bestand an mindestens 1x10⁸ verschiedenen Sequenzen von Polynucleotiden, die in stochastischer Weise synthetisiert wurden, und
(b) die Durchsicht des Bestands an Peptiden, an Polypeptiden oder an Proteinen im Hinblick auf die Bindung an den Liganden unter Bedingungen, die das Auffinden eines oder mehrerer Peptide, Polypeptide oder Proteine, die an den Liganden gebunden sind, erlauben.

2. Verfahren nach Anspruch 1, **dadurch gekennzeichnet, dass** der Schritt (a) außerdem die Synthese eines Bestands an Sequenzen von Polynucleotiden, die in stochastischer Weise hergestellt wurden, und die Übersetzung des Bestands an Sequenzen von Polynucleotiden zur Herstellung des Bestands an Peptiden, an Polypeptiden oder an Proteinen aufweist.

3. Verfahren nach Anspruch 1, **dadurch gekennzeichnet, dass** der Schritt (a) außerdem die Synthese eines Bestands an Sequenzen von Polynucleotiden, die mindestens teilweise stochastisch sind und in stochastischer Weise hergestellt wurden, und die Übersetzung des Bestands an Sequenzen von Polynucleotiden zur Herstellung des Bestands an Peptiden, an Polypeptiden oder an Proteinen aufweist.

4. Verfahren nach Anspruch 2 oder nach Anspruch 3, außerdem aufweisend die Amplifikation des Bestands an Sequenzen von Polynucleotiden, die in stochastischer Weise hergestellt wurden.

5. Verfahren nach Anspruch 1, **dadurch gekennzeichnet, dass** der Schritt (a) außerdem die Synthese eines Bestands an Sequenzen von Polynucleotiden, die in stochastischer Weise hergestellt wurden, und die Expression des Bestands an Sequenzen von Polynucleotiden zur Herstellung des Bestands an Peptiden, an Polypeptiden oder an Proteinen aufweist.

6. Verfahren nach Anspruch 1, **dadurch gekennzeichnet, dass** der Schritt (a) außerdem die Synthese eines Bestands an Sequenzen von Polynucleotiden, die mindestens teilweise stochastisch sind und in stochastischer Weise hergestellt wurden, und die Expression des Bestands an Sequenzen von Polynucleotiden zur Herstellung des Bestands an Peptiden, an Polypeptiden oder an Proteinen aufweist.

7. Verfahren nach Anspruch 1, außerdem aufweisend die Isolierung der Sequenz von Polynucleotiden, die eines oder mehrere der Peptide, Polypeptide oder Proteine, die an den Liganden binden, codiert.

8. Verfahren nach Anspruch 1, außerdem aufweisend die Verbesserung der Bindung an den Liganden durch Mutagenese in vitro oder in vivo.

9. Verfahren zur Herstellung eines Peptids, eines Polypeptids oder eines Proteins, das an einen Liganden bindet, aufweisend:
(a) Die Herstellung eines Bestands an Peptiden, an Polypeptiden oder an Proteinen, die codiert werden von einem Bestand an mindestens 1x10⁸ Sequenzen von Polynucleotiden, die in stochastischer Weise synthetisiert wurden,
(b) die Durchsicht des Bestands an Peptiden, an Polypeptiden oder an Proteinen im Hinblick auf die Bindung an den Liganden unter Bedingungen, die das Auffinden eines oder mehrerer Peptide, Polypeptide oder Proteine, die an den Liganden gebunden sind, erlauben,
(c) die Isolierung der Sequenz von Polynucleotiden, die eines oder mehrere der Peptide, Polypeptide oder Proteine, die an den Liganden binden, codiert und
(d) die Herstellung des Peptids, des Polypeptids oder des Proteins.

10. Verfahren zur Isolierung einer Sequenz von Polynucleotiden, die ein Peptid, ein Polypeptid oder ein Protein, das an einen Liganden bindet, codiert, aufweisend:
(a) Die Herstellung eines Bestands an Peptiden, an Polypeptiden oder an Proteinen, die codiert werden von einem Bestand an mindestens 1x10⁸ Sequenzen von Polynucleotiden, die in stochastischer Weise synthetisiert wurden,
(b) die Durchsicht des Bestands an Peptiden, an Polypeptiden oder an Proteinen im Hinblick auf die Bindung an den Liganden unter Bedingungen, die das Auffinden eines oder mehrerer Peptide, Polypeptide oder Proteine, die an den Liganden gebunden sind, erlauben, und
(c) die Isolierung der Sequenz oder der Sequenzen von Polynucleotiden, die das Peptid, das Polypeptid oder das Protein, das an einen Liganden bindet, codiert (codieren).

11. Verfahren zur Herstellung eines vielfältigen Bestands an Wirtszellen, aufweisend:
(a) die Synthese eines vielfältigen Bestands an in stochastischer Weise hergestellten Polynucleotid-Sequenzen, wobei der Bestand minestens 1x10⁶ unterschiedliche Polynucleotid-Sequenzen aufweist,
(b) die Insertion des vielfältigen Bestands an in stochastischer Weise hergestellten Polynucleotid-Sequenzen in einen Bestand an Vektoren, um einen vielfältigen Bestand an Vektoren, der in stochastischer Weise hergestellte Polynucleotid-Sequenzen aufweist, zu bilden, und
(c) die Einführung des vielfältigen Bestands an Vektoren in Wirtszellen.

12. Verfahren zur Identifizierung eines Polynucleotids, das an einen Liganden bindet, aufweisend:
(a) die Herstellung eines Bestands an Polynucleotiden, der mindestens 1x10⁸ unterschiedliche Polynucleotid-Sequenzen, die in stochastischer Weise synthetisiert wurden, aufweist, und
(b) die Durchsicht des Bestands an Polynucleotiden im Hinblick auf die Bindung an den Liganden unter Bedingungen, die das Auffinden eines oder mehrerer Polynucleotide, die an den Liganden binden, erlauben.

13. Verfahren nach Anspruch 12, außerdem aufweisend die Amplifikation des Bestands an Polynucleotid-Sequenzen, die in stochastischer Weise hergestellt wurden.

14. Verfahren nach Anspruch 12, **dadurch gekennzeichnet, dass** der Bestand an Polynucleotiden DNS aufweist.

15. Verfahren nach Anspruch 12, **dadurch gekennzeichnet, dass** der Bestand an Polynucleotiden RNS aufweist.

16. Verfahren nach Anspruch 12, außerdem aufweisend die Isolierung einer oder mehrerer der Polynucleotid-Sequenzen, die an den Liganden binden.

17. Verfahren nach Anspruch 12, außerdem aufweisend die Verbesserung der vorbestimmten Eigenschaft durch Mutaganese in vitro oder in vivo.

18. Verfahren zur Modifizierung der Transkription oder der Replikation einer DNS-Sequenz, aufweisend:
(a) die Herstellung eines Bestands an Peptiden, an Polypeptiden oder an Proteinen, die codiert werden von in stochastischer Weise synthetisierten Polynucleotid-Sequenzen,
(b) die Inspektion des Bestands an Peptiden, an Polypeptiden oder an Proteinen unter Bedingungen, die die Identifizierung eines oder mehrerer Peptide, Polypeptide oder Proteine, die die Transkription oder die Replikation einer DNS-Sequenz modifizieren, erlauben, und
(c) das in Kontakt bringen einer DNS-Sequenz mit einem oder mehreren der identifizierten Peptide, Polypeptide oder Proteine, die die Transkription oder die Replikation einer DNS-Sequenz modifizieren.

19. Verfahren zur Herstellung eines zur Gewinnung eines Impfstoffs brauchbaren Peptids, Polypeptids oder Proteins, folgende Schritte aufweisend:
(a) die Herstellung, durch enzymatische Kopplung, von in stochastischer Weise hergestellten Polynuoleotid-Sequenzen,
(b) die Bildung einer Bibliothek von Expressionsvektoren, die solche, in stochastischer Weise hergestellte, Polynucleotid-Sequenzen enthält,
(c) das Anlegen von Kulturen von Wirtszellen, die die Vektoren zur Herstellung von Peptiden, von Polypeptiden oder von Proteinen, die von den in stochastischer Weise hergestellten Polynucleotid-Sequenzen codiert werden, enthalten,
(d) die Durchführung der Durchsicht oder der Auswahl von Wirtszellen dieser Art, um ein durch die Wirtszellen hergestelltes Peptid, Polypeptid oder Protein mit mindestens einem Epitop, das einer AminosäureSequenz eines der Epitope eines gegebenen Antigens ähnlich ist, zu identifizieren,
(e) die Isolierung einer in stochastischer Weise hergestellten Polynucleotid-Sequenz, die das identifizierte Peptid, Polypeptid oder Protein codiert und
(f) die Verwendung der isolierten Sequenz zur Herstellung des Peptids, des Polypeptids oder des Proteins mit der Eigenschaft.
